# EUROPÄISCHE PATENTSCHRIFT

(11) **EP 1 280 792 B1**
(45) Veröffentlichungstag und Bekanntmachung des Hinweises auf die Patenterteilung: **14.07.2004**
(21) Anmeldenummer: 01921324.8
(22) Anmeldetag: 09.03.2001
(51) Int. Cl.: C07D 333/68

(54) **VERFAHREN ZUR HERSTELLUNG BENZO-ANNELIERTER HETEROCYCLEN**
METHOD FOR PRODUCING BENZO ANNELATED HETEROCYCLES
PROCEDE DE PRODUCTION D'HETEROCYCLES BENZO-ANNELES

(30) Priorität: 11.04.2000 DE 10017947
(43) Veröffentlichungstag der Anmeldung: 05.02.2003
(73) Patentinhaber: MERCK PATENT GmbH, 64293 Darmstadt (DE)
(72) Erfinder: WARTENBERG, Frank-Hardi, 64289 Darmstadt (DE); KOPPE, Thomas, CH-8200 Schaffhausen (CH); WETZEL, Walter, 64291 Darmstadt (DE); WYDRA, Markus, 63322 Rödermark (DE); BENZ, Achim, 64291 Darmstadt (DE)
(86) Internationale Anmeldenummer: PCT/EP2001/002672
(87) Internationale Veröffentlichungsnummer: WO 2001/077099

(56) Entgegenhaltungen:
- WO-A-99/55708
- US-A- 4 888 432

## Beschreibung

Die Erfindung betrifft ein Verfahren zur Herstellung von benzo-annelierten Heterocyclen der allgemeinen Formel I 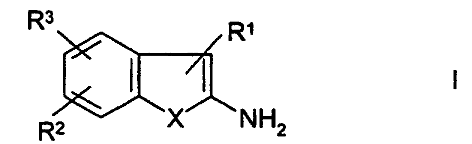 worin
- X: S, O oder NH,
- R¹: CN, NO₂, Ac, COAr, COOAr, COOH, COOA oder CONR⁴R⁵,
- R² und R³: jeweils unabhängig voneinander H, A, NO₂, CN, OH, OA oder Ac,
- R⁴ und R⁵: jeweils unabhängig voneinander H, A, Ar oder Ac,
- R⁴ und R⁵: zusammen auch -(CH₂)-(CH₂)ₙ-(CH₂)-,
- A: Alkyl mit 1-6 C-Atomen,
- Ac: Acyl mit 1-6 C-Atomen,
- Ar: unsubstituiertes oder durch A, NO₂, CN, OH, OA substituiertes Phenyl,
- n: 2, 3 oder 4
bedeutet,
durch Umsetzung von tetrahydrobenzo-annelierten Heterocyclen der Formel II 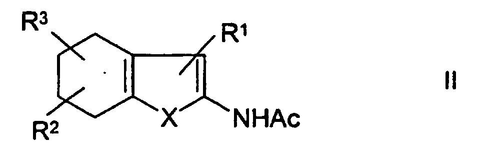 worin
- X: S, O oder NH,
- R¹: CN, NO₂, Ac, COAr, COOAr, COOH, COOA oder CONR⁴R⁵,
- R² und R³: jeweils unabhängig voneinander H, A, NO₂, CN, OH, OA oder Ac,
- R⁴ und R⁵: jeweils unabhängig voneinander H, A, Ar oder Ac,
- R⁴ und R⁵: zusammen auch -(CH₂)-(CH₂)ₙ-(CH₂)-,
- A: Alkyl mit 1-6 C-Atomen,
- Ac: Acyl mit 1-6 C-Atomen,
- Ar: unsubstituiertes oder durch A, NO₂, CN, OH oder OA substituiertes Phenyl,
- n: 2, 3 oder 4
bedeutet,
mit einer katalytischen Menge eines Edelmetallkatalysators in Gegenwart eines Wasserstoffakzeptors und anschließender Deacylierung der acylierten Aminogruppe durch Zugabe eines Amins.

Benzo-annelierte Heterocyclen der Formel I sind wichtige Zwischenstufen in der großtechnischen organischen Synthese, z.B. bei der Herstellung von Feinchemikalien, Farbstoffen und Pflanzenschutzmitteln. Sie sind weiterhin wichtige Zwischenstufen bei der Herstellung von Arzneimitteln. Benzo-annelierte Heterocyclen der Formel I, worin X S bedeutet, sind insbesondere bei der Herstellung von PDE-V-Hemmem, die aus WO 99/55708 und WO 00/78767 bekannt sind, von Bedeutung. Insbesondere 2-Amino-benzo[b]thiophen-3-carbonsäureethylester ist ein Zwischenprodukt bei der Synthese von 4-[4-(3-Chlor-4-methoxybenzylamino)-benzo[4,5]thieno[2,3-d]pyrimidin-2-yl]-cyclohexancarbonsäure, bekannt aus WO 99/55708 oder von 4-[4-(3-Chlor-4-hydroxy-benzylamino)-benzo[4,5]thieno[2,3-d]pyrimidin-2-yl]-cyclohexancarbonsäure, bekannt aus WO 00/78767.

Eine Aromatisierung von tetrahydrobenzo-annelierten Verbindungen wird nach der klassischen Synthese durch Umsetzungen mit elementarem Schwefel bei hohen Temperaturen durchgeführt (Literatur: Gewald et al., Chem. Ber. 1968, 101, 1933). Nachteile dieses Verfahrens sind die hohen Energiekosten, verursacht durch hohe Reaktionstemperaturen, die Freisetzung von geruchsbelästigendem Schwefelwasserstoff und die auftretende Problematik bei der Reinigung, da sich elementarer Schwefel nur im sehr leicht entflammbaren CS₂ löst.
Ein spezielles Beispiel aus dem Stand der Technik ist die Umsetzung der Verbindung 2-Acetylamino-3-methoxy-carbonyl-4,5-tetramethylen-thiophen mit 2 Äquivalenten Schwefel und Dimethylphthalat bei Temperaturen zwischen 200-220°C nach G. Hallas et al., Dyes Pigm. 1997, 35, 219-237.
Das isolierte 2-Acetylamino-3-methoxycarbonylbenzo[b]thiophen wird anschließend in einer zweiten Stufe durch Reaktion mit einer wäßrigen Kaliumhydroxidlösung in Ethanol deacetyliert.

Eine weitere bekannte Möglichkeit der Aromatisierung ist die Reaktion einer tetrahydrobenzo-annelierten Verbindung mit einer äquimolaren Menge eines Hydrierungskatalysators. Ein spezielles Beispiel, die Dehydrierung von 2-Acetylamino-tetrahydrobenzothiophen-3-carbonsäuremethylester mit einer annähernd äquimolaren Menge an Palladium auf Kohle (10% Pd/C) im Lösungsmittel Chloroform, wird in Eiden et al., Arch. Pharm. 1984, 317, 675-680 beschrieben.

Aus ökologischen Gründen ist die Umsetzung mit elementarem Schwefel für eine großtechnische Anwendung unpraktikabel.

Bei der zweiten Variante sollte aus ökonomischen Gründen die Menge an eingesetztem Hydrierkatalysator so gering wie möglich gehalten werden. Die bei der Dehydrierung entstehenden benzo-annelierten Heterocyclen sind weiterhin in den verwendeten Lösungsmitteln häufig schwerlöslich und fallen beim Abkühlen des heterogenen Reaktionsgemisches aus. Dadurch wird eine Abtrennung des Edelmetallkatalysators erschwert und es werden erhebliche Mengen Lösungsmittel zur Extraktion des Produktes aus dem Edelmetallkatalysator notwendig.

Der Erfindung lag daher die Aufgabe zugrunde, ein Verfahren zur Herstellung von benzo-annelierten Heterocylen der Formel I zu entwickeln, welches Vorteile gegenüber den bekannten Verfahren des Standes der Technik besitzt.

Überraschenderweise wurde gefunden, daß eine Aromatisierung von tetrahydrobenzo-annelierten Verbindungen der Formel II mit einer katalytischen Menge eines Hydrierkatalysators in Gegenwart eines Wasserstoffakzeptors ermöglicht wird. Die sich direkt anschließende Deacylierung der Aminogruppe in 2-Position des Heterocyclus durch Zugabe eines Amins stellt die benzo-annelierten Verbindungen der Formel I als gut lösliche Produkte bereit, so daß der Edelmetallkatalysator durch eine einfache Filtration abgetrennt werden kann. Das erfindungsgemäße Verfahren ist ein Eintopf-Verfahren, d.h. Aromatisierung und Deacylierung finden nacheinander statt, ohne das Zwischenprodukt, in diesem Fall den benzo-annelierten Heterocyclus, dessen Aminogruppe acyliert vorliegt, zu isolieren.

Für alle Reste, die mehrfach auftreten, wie z.B. A oder Ac, gilt, daß deren Bedeutungen unabhängig voneinander sind.

Der Rest A bedeutet Alkyl und hat 1 bis 6 , vorzugsweise 1, 2, 3 oder 4, insbesondere 1 oder 2 C-Atome. Alkyl bedeutet daher insbesondere z.B. Methyl, weiterhin Ethyl, n-Propyl, Isopropyl, n-Butyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, 1-, 2-, 3- oder 4-Methylpentyl, 1,1-, 1,2-, 1,3-, 2,2-, 2,3- oder 3,3-Dimethylbutyl, 1- oder 2-Ethylbutyl, 1-Ethyl-1-methylpropyl, 1-Ethyl-2-methylpropyl, 1,1,2- oder 1,2,2-Trimethylpropyl. Besonders bevorzugt ist A Methyl oder Ethyl.

Ac bedeutet Acyl und hat vorzugsweise 1-6 C-Atome. Ac bedeutet beispielsweise Formyl, Acetyl, Propionyl, Butyryl, Pentanoyl oder Hexanoyl, ferner Trifluoracetyl. Besonders bevorzugt ist Ac Acetyl.

Ar bedeutet unsubstituiertes oder durch A, NO₂, CN, OH oder OA substituiertes Phenyl.

Ar bedeutet daher bevorzugt Phenyl, o-, m- oder p-Methylphenyl, o-, m-oder p-Ethylphenyl, o-, m- oder p-Propylphenyl, o-, m- oder p-Isopropylphenyl, o-, m- oder p-tert.-Butylphenyl, o-, m- oder p-Hydroxyphenyl, o-, m- oder p-Methoxyphenyl, o-, m- oder p-Ethoxyphenyl, o-, m-, p-Nitrophenyl oder o-, m- oder p-Cyanophenyl. Besonders bevorzugt ist Ar unsubstituiertes Phenyl.

COAr bedeutet Aroyl, wobei Ar eine der zuvor angegebenen Bedeutungen hat. Besonders bevorzugt für COAr ist Benzoyl.

COOAr bedeutet Aryloxycarbonyl, wobei Ar eine der zuvor angegebenen Bedeutungen hat. Besonders bevorzugt für COOAr ist Phenoxycarbonyl.

X bedeutet S, O oder NH. S ist für X besonders bevorzugt.

R¹ bedeutet CN, NO₂, Ac, COAr, COOAr, COOH, COOA oder CONR⁴R⁵, wobei A, Ac und Ar eine der zuvor angegebenen Bedeutungen haben und R⁴ und R⁵ eine der nachstehend angegebenen Bedeutungen haben. Besonders bevorzugt ist R¹ = CN oder COOA, ganz besonders bevorzugt COOA.

R² und R³ bedeuten jeweils unabhängig voneinander H, A, NO₂, CN, OH, OA oder Ac, wobei A und Ac eine der zuvor angegebenen Bedeutungen haben. Besonders bevorzugt ist R² = H und R³ = H.

R⁴ und R⁵ bedeuten jeweils unabhängig voneinander H, A, Ar oder Ac, wobei A, Ar und Ac eine der zuvor angegebenen Bedeutungen haben. Besonders bevorzugt ist R⁴ = H und R⁵ = H.
R⁴ und R⁵ bedeuten zusammen auch -(CH₂)-(CH₂)ₙ-(CH₂)-, wobei n 2, 3 oder 4 sein kann. Besonders bevorzugt sind R⁴ und R⁵ zusammen -(CH₂)-(CH₂)₂-(CH₂)- oder -(CH₂)-(CH₂)₃-(CH₂)-, ganz besonders bevorzugt -(CH₂)-(CH₂)₃-(CH₂)-.

Als Hydrierkatalysatoren, oder auch synonym dazu Edelmetallkatalysatoren, können Edelmetalle wie Palladium, Platin oder Rhodium eingesetzt werden, die auf einem geeigneten Trägermaterial bereitgestellt werden. Geeignete Trägermaterialien sind Kohle, Aktivkohle, Aluminiumoxid, Bariumcarbonat, Bariumsulfat, Calciumcarbonat oder Strontiumcarbonat. Der prozentuale Anteil des Edelmetalls im Edelmetallkatalysator liegt zwischen 1 und 20 %, bevorzugt zwischen 5 und 10%, besonders bevorzugt bei 5%.
Für das erfindungsgemäße Verfahren können insbesondere Palladium auf Aktivkohle, Kohle, Aluminiumoxid, Bariumcarbonat, Bariumsulfat, Calciumcarbonat oder Strontiumcarbonat, Platin auf Aktivkohle, Kohle oder Aluminiumoxid oder Rhodium auf Kohle oder Aluminiumoxid, eingesetzt werden. Besonders bevorzugt wird Palladium auf Aktivkohle (5% Pd) eingesetzt.

Es können ferner Salze der Edelmetalle eingesetzt werden, die in situ durch ein Reduktionsmittel reduziert werden können und in situ eine fein verteilte Palladium(0)-Spezies erzeugen. Geeignete Edelmetallsalze sind beispielsweise Palladiumacetat, Palladiumbromid oder Palladiumchlorid, geeignete Reduktionsmittel sind beispielsweise Wasserstoff, Hydrazin, Natriumborhydrid oder Formiate.

Gegenstand der Erfindung ist ferner ein Verfahren zur Herstellung benzo-annelierter Heterocyclen der allgemeinen Formel I nach Anspruch 1 oder 2, dadurch gekennzeichnet, daß ein Edelmetallkatalysator, ausgewählt aus der Gruppe Palladium auf Aktivkohle, Kohle, Aluminiumoxid, Bariumcarbonat, Bariumsulfat, Calciumcarbonat oder Strontiumcarbonat, Platin auf Aktivkohle, Kohle oder Aluminiumoxid oder Rhodium auf Kohle oder Aluminiumoxid, eingesetzt wird.

Für das erfindungsgemäße Verfahren sind insbesondere wohlfeile, organische Wasserstoffakzeptoren geeignet, wie sie dem Fachmann bekannt sind. Beispiele für wohlfeile organische Wasserstoffakzeptoren sind Styrol, α-Methylstyrol, Stilben, Tolane, Zimtsäureester oder Cyclohexen. Besonders bevorzugt wird α-Methylstyrol eingesetzt. Für das erfindungsgemäße Verfahren ebenfalls geeignete Wasserstoffakzeptoren sind Sauerstoff oder Sauerstoff-Gas-Gemische, wobei unter Gas Stickstoff oder Edelgase wie Helium, Neon, Argon oder Xenon verstanden werden. Der prozentuale Anteil des Sauerstoffs im Sauerstoff-Gas-Gemisch liegt zwischen 1 und 99%, vorzugsweise zwischen 10 und 50 %, besonders bevorzugt bei 15 bis 25%. Ein besonders bevorzugtes Sauerstoff-Gas-Gemisch ist Luft.

Es ist während der Aromatisierung möglich, daß Polymerisationsreaktionen des organischen Wasserstoffakzeptors stattfinden. Die Entstehung dieser Nebenprodukte, d.h. der Polymere, kann vermindert werden, in dem der Wasserstoffakzeptor in kleinen Mengen nacheinander (halbkontinuierlich) oder kontinuierlich während der Reaktion zugegeben wird.

Gegenstand der Erfindung ist ebenfalls ein Verfahren zur Herstellung benzo-annelierter Heterocyclen der allgemeinen Formel I nach einem oder mehreren der Ansprüche 1 bis 3, dadurch gekennzeichnet, daß ein Wasserstoffakzeptor, ausgewählt aus der Gruppe Styrol, α-Methylstyrol, Stilben, Tolane, Zimtsäureester, beispielsweise Zimtsäuremethyl- oder Zimtsäureethylester, Cyclohexen, Sauerstoff oder Sauerstoff-Gas-Gemisch, eingesetzt wird.

Die Dehydrierung - und damit die Aromatisierung - unter Verwendung einer katalytischen Menge eines Hydrierkatalysators sollte vorteilhafter Weise unter Inertgasatmosphäre durchgeführt werden, um Explosionen zu vermeiden. Daher ist der Einsatz der organischen Wasserstoffakzeptoren, ausgewählt aus der Gruppe Styrol, α-Methylstyrol, Stilben, Tolane, Zimtsäureester oder Cyclohexen von Vorteil.

Zur Deacylierung der acylierten Aminogruppe der Verbindungen der allgemeinen Formel II sowie des benzo-annelierten Zwischenprodukts nach Aromatisierung wird dem Reaktionsgemisch ein primäres oder sekundäres Amin zugegeben, dessen Siedepunkt zwischen 50 und 200°C, vorzugsweise zwischen 50 und 150°C liegt. Besonders bevorzugt werden Pyrrolidin, Piperidin, Piperazin, Morpholin oder Dioctylamin eingesetzt. Ganz besonders bevorzugt wird Pyrrolidin eingesetzt.

Gegenstand der Erfindung ist ein Verfahren, wie zuvor beschrieben, dadurch gekennzeichnet, daß zur Deacylierung ein primäres oder sekundäres Amin ausgewählt wird, dessen Siedepunkt zwischen 50 und 200°C liegt.

Die Aromatisierung sowie die Deacylierung finden bevorzugt in einem inerten, hochsiedenden Lösungsmittel statt. Bevorzugte inerte, hochsiedende Lösungsmittel sind Benzol, Toluol, Xylol, Mesitylen, Diphenylether oder Sulfolan. Xylol wird bevorzugt als Isomerengemisch eingesetzt. Die Isomeren o-, m- oder p-Xylol sind ebenfalls geeignet. Besonders bevorzugt wird Xylol eingesetzt.

Gegenstand der Erfindung ist das zuvor beschriebene Verfahren, dadurch gekennzeichnet, daß die Reaktionen in einem inerten, hochsiedendem Lösungsmittel durchgeführt werden.

Die Dehydrierung sowie die Deacylierung finden bevorzugt bei Temperaturen zwischen 50° und 250°C statt. Der bevorzugte Temperaturbereich für die Dehydrierung liegt zwischen 100° und 250°C, besonders bevorzugt zwischen 140 und 200°C. Bevorzugter Temperaturbereich für die Deacylierung ist zwischen 50° und 200°C, besonders bevorzugt zwischen 80° und 150°C.

Gegenstand der Erfindung ist das zuvor beschriebene Verfahren, dadurch gekennzeichnet, daß die Reaktionen bei Temperaturen zwischen 50 und 200°C durchgeführt werden.

Entsprechend dem erfindungsgemäßen Verfahren liegen die Ausbeuten an benzo-annelierten Heterocyclen der Formel I in der Regel zwischen 65% und 80%, einschließlich des Deacylierungsschrittes.

Besonders eignet sich das erfindungsgemäße Verfahren, wie zuvor beschrieben, zur Herstellung von 2-Amino-benzo[b]thiophen-3-carbonsäuremethyl- oder -ethylester. Erfindungsgemäß wird dafür 2-Acetylamino-tetrahydrobenzothiophen-3-carbonsäuremethyl- oder -ethylester mit einer katalytischen Menge eines Edelmetallkatalysators in Gegenwart eines Wasserstoffakzeptors umgesetzt und anschließend die acetylierte Aminogruppe durch Zugabe eines Amins deacetyliert.

Gegenstand der Erfindung ist ferner die Verwendung von 2-Aminobenzo[b]thiophen-3-carbonsäuremethyl- oder -ethylester, hergestellt nach dem zuvor beschriebenen Verfahren, als Zwischenprodukt bei der Synthese von 4-[4-(3-Chlor-4-methoxy-benzylamino)-benzo[4,5]thieno[2,3-d]pyrimidin-2-yl]-cyclohexancarbonsäure, bekannt aus WO 99/55708. Weitere Zwischenprodukte der Synthese von 4-[4-(3-Chlor-4-methoxybenzylamino)-benzo[4,5]thieno[2,3-d]pyrimidin-2-yl]-cyclohexancarbonsäure, ausgehend von dem entsprechenden 2-Aminobenzo[b]thiophen-3-carbonsäuremethylester, sind 4-(4-Hydroxybenzo[4,5]thieno[2,3-d]pyrimidin-2-yl)-cyclohexancarbonsäuremethylester, 4-(4-Chlor-benzo[4,5]thieno[2,3-d]pyrimidin-2-yl)-cyclohexancarbonsäuremethylester und 4-[4-(3-Chlor-4-methoxybenzylamino)-benzo[4,5]thieno[2,3-d]pyrimidin-2-yl]-cyclohexancarbonsäuremethylester.

In dem aus WO 99/55708 bekannten Verfahren zur Herstellung von 4-[4-(3-Chlor-4-methoxy-benzylamino)-benzo[4,5]thieno[2,3-d]pyrimidin-2-yl]-cyclohexancarbonsäure wird in der
Stufe a) 2-Amino-tetrahydrobenzothiophen-3-carbonsäuremethylester mit 4-Cyano-cyclohexancarbonsäuremethylester zunächst cyclisiert, in
Stufe b) die Tetrahydrobenzothiophen-Einheit des entstandenen Zwischenprodukts mit Schwefel zu der Verbindung 4-(4-Hydroxybenzo[4,5]thieno[2,3-d]pyrimidin-2-yl)-cyclohexancarbonsäuremethylester dehydriert, in
Stufe c) durch Umsetzung mit einem Chlorierungsmittel, vorzugsweise POCl₃, die Hydroxygruppe chloriert, in
Stufe d) die Verbindung 4-(4-Chlor-benzo[4,5]thieno[2,3-d]pyrimidin-2-yl)-cyclohexancarbonsäuremethylester aus Stufe c) mit 3-Chlor-4-methoxybenzylamin zu dem Ester 4-[4-(3-Chlor-4-methoxybenzylamino)-benzo[4,5]thieno[2,3-d]pyrimidin-2-yl)-cyclohexancarbonsäuremethylester umgesetzt und in
Stufe e) den Ester aus Stufe d) verseift und in
Stufe f) die freie Säure in ein pharmakologisch unbedenkliches Salz überführt.

Die Umsetzung mit elementarem Schwefel ist jedoch aus ökologischen Gründen für eine großtechnische Anwendung unpraktikabel.

Gegenstand der Erfindung ist daher ein Verfahren zur Herstellung von 4-[4-(3-Chlor-4-methoxy-benzylamino)-benzo[4,5]thieno[2,3-d]pyrimidin-2-yl]-cyclohexancarbonsäure oder einem der pharmazeutisch unbedenklichen Salze, dadurch gekennzeichnet, daß man in
Stufe a) 2-Acetylamino-tetrahydrobenzothiophen-3-carbonsäurealkylester gemäß einem oder mehreren der Ansprüche 1 bis 7 mit einer katalytischen Menge eines Edelmetallkatalysators in Gegenwart eines Wasserstoffakzeptors umsetzt und anschließend die acetylierte Aminogruppe durch Zugabe eines Amins zur Verbindung von 2-Aminobenzo[b]thiophen-3-carbonsäurealkylester deacetyliert, in
Stufe b) 2-Amino-benzo[b]thiophen-3-carbonsäurealkylester durch Reaktion mit 4-Cyano-cyclohexancarbonsäurealkylester cyclisiert, in
Stufe c) die Hydroxygruppe der Verbindung 4-(4-Hydroxybenzo[4,5]thieno[2,3-d]pyrimidin-2-yl)-cyclohexancarbonsäurealkylester aus Stufe b) mit einem Chlorierungsmittel chloriert, in
Stufe d) die Verbindung 4-(4-Chlor-benzo[4,5]thieno[2,3-d]pyrimidin-2-yl)-cyclohexancarbonsäurealkylester aus Stufe c) mit 3-Chlor-4-methoxybenzylamin zu dem Ester 4-[4-(3-Chlor-4-methoxybenzylamino)-benzo[4,5]thieno[2,3-d]pyrimidin-2-yl]-cyclohexancarbonsäurealkylester umsetzt, in
Stufe e) den Ester aus Stufe d) verseift und in
Stufe f) die freie Säure in ein pharmakologisch unbedenkliches Salz überführt.

Alkylester bedeutet beispielsweise Methylester, Ethylester, Propylester oder Butylester. Bevorzugt wird für 2-Acetylaminotetrahydrobenzothiophen-carbonsäurealkylester und alle nachfolgenden Zwischenprodukte, basierend auf dieser Estereinheit, 2-Acetylaminotetrahydrobenzothiophen-3-carbonsäuremethyl- oder 2-Acetylaminotetrahydrobenzothiophen-3-carbonsäureethylester eingesetzt.
Bevorzugt wird für 4-Cyano-cyclohexancarbonsäurealkylester und alle nachfolgenden Zwischenprodukte, basierend auf dieser Estereinheit, trans-4-Cyano-cyclohexancarbonsäuremethylester eingesetzt.

Die Reaktionsbedingungen der Cyclisierung b) bei der erfindungsgemäßen Herstellung von 4-[4-(3-Chlor-4-methoxy-benzylamino)-benzo[4,5]thieno[2,3-d]pyrimidin-2-yl]-cyclohexancarbonsäure sind aus Eur. J. Med. Chem. 1988, 23, 453 bekannt.

Geeignete Chlorierungsmittel sind beispielsweise POCl₃, SOCl₂ oder Cyanurchlorid. Die Chlorierung mit POCl₃ oder SOCl₂ findet unter Reaktionsbedingungen statt, die für den Fachmann bekannt sind. Bevorzugt findet die Reaktion in dem inerten Lösungsmittel, beispielsweise in Toluol, Methylenchlorid oder Dimethylformamid statt.

Die Umsetzungen von 4-(4-Chlor-benzo[4,5]thieno[2,3-d]pyrimidin-2-yl)-cyclohexancarbonsäuremethylester in Stufe d) und e) des erfindungsgemäßen Verfahrens zur Herstellung von 4-[4-(3-Chlor-4-methoxy-benzylamino)-benzo[4,5]thieno[2,3-d]pyrimidin-2-yl]-cyclohexancarbonsäure, wie zuvor beschrieben, und die Stufe f) sind aus WO 99/55708, Beispiel 1 S. 11-12 und Beispiel 2 S. 14 bekannt.

Gegenstand der Erfindung ist ferner die Verwendung von 2-Aminobenzo[b]thiophen-3-carbonsäuremethyl- oder -ethylester, hergestellt nach dem zuvor beschriebenen Verfahren, als Zwischenprodukt bei der Synthese von 4-[4-(3-Chlor-4-hydroxy-benzylamino)-benzo[4,5]thieno[2,3-d]pyrimidin-2-yl]-cyclohexancarbonsäure, bekannt aus WO 00/78767. Weitere Zwischenprodukte der Synthese von 4-[4-(3-Chlor-4-hydroxybenzylamino)-benzo[4,5]thieno[2,3-d]pyrimidin-2-yl]-cyclohexancarbonsäure, ausgehend von dem entsprechenden 2-Aminobenzo[b]thiophen-3-carbonsäuremethylester, sind 4-(4-Hydroxybenzo[4,5]thieno[2,3-d]pyrimidin-2-yl)-cyclohexancarbonsäuremethylester, 4-(4-Chlor-benzo[4,5]thieno[2,3-d]pyrimidin-2-yl)-cyclohexancarbonsäuremethylester und 4-[4-(3-Chlor-4-hydroxybenzylamino)-benzo[4,5]thieno[2,3-d]pyrimidin-2-yl]-cyclohexancarbonsäuremethylester.

In dem aus WO 00/78767 bekannten Verfahren zur Herstellung von 4-[4-(3-Chlor-4-hydroxy-benzylamino)-benzo[4,5]thieno[2,3-d]pyrimidin-2-yl]-cyclohexancarbonsäure wird 4-(4-Chlor-benzothieno-[2,3-d]-pyrimidin-2-yl)-cyclohexancarbonsäuremethylester mit 3-Chlor-4-hydroxy-benzylamin umgesetzt, wobei 4-(4-Chlor-benzothieno-[2,3-d]-pyrimidin-2-yl)-cyclohexancarbonsäuremethylester, wie ebenfalls in WO 99/55708 beschrieben, durch Cyclisierung von 2-Amino-5,6,7,8-tetrahydrobenzothiophen-3-carbonsäuremethylester mit 3-Cyancyclohexancarbonsäuremethylester, Dehydrierung mit Schwefel und nachfolgender Chlorierung mit Phosphoroxichlorid/Dimethylamin hergestellt wird.

Die Umsetzung mit elementarem Schwefel ist jedoch aus ökologischen Gründen für eine großtechnische Anwendung unpraktikabel.

Gegenstand der Erfindung ist daher ein Verfahren zur Herstellung von 4-[4-(3-Chlor-4-hydroxy-benzylamino)-benzo[4,5]thieno[2,3-d]pyrimidin-2-yl]-cyclohexancarbonsäure oder einem der pharmazeutisch unbedenklichen Salze, dadurch gekennzeichnet, daß man in
Stufe a) 2-Acetylamino-tetrahydrobenzothiophen-3-carbonsäurealkylester gemäß einem oder mehreren der Ansprüche 1 bis 7 mit einer katalytischen Menge eines Edelmetallkatalysators in Gegenwart eines Wasserstoffakzeptors umsetzt und anschließend die acetylierte Aminogruppe durch Zugabe eines Amins zur Verbindung von 2-Aminobenzo[b]thiophen-3-carbonsäurealkylester deacetyliert, in
Stufe b) 2-Amino-benzo[b]thiophen-3-carbonsäurealkylester durch Reaktion mit 4-Cyano-cyclohexancarbonsäurealkylester cyclisiert, in
Stufe c) die Hydroxygruppe der Verbindung 4-(4-Hydroxybenzo[4,5]thieno[2,3-d]pyrimidin-2-yl)-cyclohexancarbonsäurealkylester aus Stufe b) mit einem Chlorierungsmittel chloriert, in
Stufe d) die Verbindung 4-(4-Chlor-benzo[4,5]thieno[2,3-d]pyrimidin-2-yl)-cyclohexancarbonsäurealkylester aus Stufe c) mit 3-Chlor-4-hydroxybenzylamin zu dem Ester 4-[4-(3-Chlor-4-hydroxybenzylamino)-benzo[4,5]thieno[2,3-d]pyrimidin-2-yl]-cyclohexancarbonsäurealkylester umsetzt, in
Stufe e) den Ester aus Stufe d) verseift und in
Stufe f) die freie Säure in ein pharmakologisch unbedenkliches Salz überführt.

Die Definitionen des Begriffes Alkylester und die Reaktionsbedingungen der Cyclisierung und Chlorierung, wie zuvor für 4-[4-(3-Chlor-4-methoxybenzylamino)-benzo[4,5]thieno[2,3-d]pyrimidin-2-yl]-cyclohexancarbonsäure beschrieben, gelten auch für die Verbindung 4-[4-(3-Chlor-4-hydroxy-benzylamino)-benzo[4,5]thieno[2,3-d]pyrimidin-2-yl]-cyclohexancarbonsäure. Die Reaktionsbedingungen der Schritte d) bis f) sind aus WO 99/55708, Beispiele 1 und 2 sowie aus WO 00/78767, Beispiele 1 und 7 bekannt.

Bei den nachfolgenden Beispielen und auch bei den vorherigen Ausführungen wird die Temperatur in °C angegeben. In den Beispielen bedeutet "übliche Aufarbeitung": Man gibt, falls erforderlich, Wasser hinzu, stellt, falls erforderlich, je nach Konstitution des Endprodukts auf pH-Werte zwischen 2 und 10 ein, extrahiert mit Ethylacetat oder Dichlormethan, trennt ab, trocknet die organische Phase über Natriumsulfat, dampft ein und reinigt durch Chromatographie an Kieselgel und/oder durch Kristallisation.

### Beispiel 1:

Eine Suspension von 22,9 g Palladium auf Aktivkohle Pd/C 5% (Degussa-Hüls; E 101 Rw 5%, 53.9% wasserfeucht) in 260 ml Xylol wird am Wasserabscheider so lange zum Rückfluß erhitzt, bis sich kein Wasser mehr abscheidet. Zu diesem Reaktionsgemisch wird bei Raumtemperatur eine Lösung von 49,7 g N-Acetylthiophennitril in 210 ml Xylol zugegeben und das Reaktionsgemisch auf 143°C erhitzt. Nach 2 h werden 48 g α-Methylstyrol, nach 96 h weitere 25 g α-Methylstyrol zugegeben. Durch Zugabe von 47 g Pyrrolidin nach 127 h Reaktionszeit wird die Deacylierung eingeleitet, wobei die Temperatur bei 137°C gehalten wird. Nach 48 h Reaktionszeit wird heiß filtriert und der Rückstand mit 100 ml Essigsäureethylester und 200 ml einer 10%igen HCl-Lösung nachgewaschen. Die organische Phase wird so lange mit destilliertem Wasser gewaschen, bis das Waschwasser einen pH-Wert 4 hat. Das organische Lösungsmittel wird abdestilliert und man erhält 2-Aminobenzo[b]thiophen-3-carbonitril in einer Ausbeute von 81%.

### Beispiel 2:

Zu einer Lösung von 10 g 2-Acetylamino-tetrahydrobenzothiophencarbonsäureethylester in 80 ml Mesitylen werden 2 g Palladiumkatalysator (Pd/C 5 %, ca. 50 % wasserfeucht; Degussa-Hüls; E 101 RW 5%) zugegeben und unter Stickstoff auf 170° erhitzt. Innerhalb von 30 min werden 2 g Ethylzinnamat zudosiert und 21 h weiter gerührt. Danach wird die Temperatur auf 100° gesenkt und 10 ml Pyrrolidin zugegeben. Das Reaktionsgemisch wird 25 h unter Stickstoff gerührt. Anschließend wird der Katalysator abfiltriert und mit 60 g Ethanol nachgewaschen. Die Lösungen werden vereint und das Lösungsmittel abdestilliert. Der Rückstand wird in Essigsäureethylester aufgenommen, zweimal mit 1N HCl und einmal mit Wasser gewaschen. Man erhält nach Abdestillieren des Lösungsmittels und anschließender Kristallisation aus 2-Propanol 2-Aminobenzo[b]thiophen-3-carbonsäureethylester mit einer Ausbeute von 64 %.

### Beispiel 3:

1. Zu einer Lösung von 36 g 2-Acetylamino-tetrahydrobenzothiophen-3-carbonsäureethylester in 250ml Xylol (Isomerengemisch) werden 14,4 g Hydrierkatalysator (Degussa-Hüls; E 101 R/W5%) zugegeben und auf 139°C bis 141 °C temperiert. Über einen Zeitraum von 5 h werden 47 g α-Methylstyrol zudosiert. Anschließend wird noch 36 h refluxiert. Es wird auf 102°C abgekühlt, 32 g Pyrrolidin zugegeben und wieder zum reflux gebracht (127°C); bei dieser Temperatur wird 20 h gerührt. Nach Abkühlen auf 20°C wird von der Pd-Kohle abfiltriert, das Filtrat zum Rückstand eingeengt, in 75 ml Ethylacetat aufgenommen und dreimal mit je 12 ml 10% Salzsäure gewaschen. Die organische Phase wird zweimal mit jeweils 5 ml Wasser gewaschen, dann zum Rückstand eingeengt. Dieser wird aus 50 ml Isopropanol umkristallisiert und liefert 21 g 2-Amino-benzothiophen-3-carbonsäureethylester als leicht gelbliche Kristalle (70.5% Ausbeute).

Die weiteren Syntheseschritte sind aus Eur. J. Med. Chem. 1988, 23, 453 und WO 99/55708, Beispiel 1 S. 11-12 und Beispiel 2 S. 14 bekannt.

## Patentansprüche

1. Verfahren zur Herstellung benzo-annelierter Heterocyclen der allgemeinen Formel I 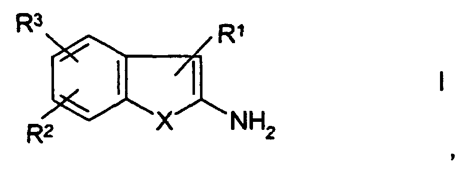 worin
X S, O oder NH,
R¹ CN, NO₂, Ac, COAr, COOAr, COOH, COOA oder CONR⁴R⁵,
R² und R³ jeweils unabhängig voneinander H, A, NO₂, CN, OH, OA oder Ac,
R⁴ und R⁵ jeweils unabhängig voneinander H, A, Ar oder Ac,
R⁴ und R⁵ zusammen auch -(CH₂)-(CH₂)ₙ-(CH₂)-,
A Alkyl mit 1-6 C-Atomen,
Ac Acyl mit 1-6 C-Atomen,
Ar unsubstituiertes oder durch A, NO₂, CN, OH oder OA substituiertes Phenyl,
n 2, 3 oder 4
bedeutet,
durch Umsetzung von tetrahydrobenzo-annelierten Heterocyclen der Formel II 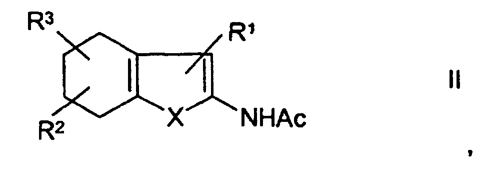 worin
X S, O oder NH,
R¹ CN, NO₂, Ac, COAr, COOAr, COOH, COOA oder CONR⁴R⁵,
R² und R³ jeweils unabhängig voneinander H, A, NO₂, CN, OH, OA oder Ac,
R⁴ und R⁵ jeweils unabhängig voneinander H, A, Ar oder Ac,
R⁴ und R⁵ zusammen auch -(CH₂)-(CH₂)ₙ-(CH₂)-,
A Alkyl mit 1-6 C-Atomen,
Ac Acyl mit 1-6 C-Atomen,
Ar unsubstituiertes oder durch A, NO₂, CN, OH, OA substituiertes Phenyl,
n 2, 3 oder 4
bedeutet,
mit einer katalytischen Menge eines Edelmetallkatalysators in Gegenwart eines Wasserstoffakzeptors und anschließender Deacylierung der acylierten Aminogruppe durch Zugabe eines Amins.

2. Verfahren gemäß Anspruch 1 zur Herstellung von 2-Aminobenzo[b]thiophen-3-carbonsäuremethyl- oder 2-Aminobenzo[b]thiophen-3-carbonsäureethyl-ester.

3. Verfahren nach Anspruch 1 oder 2, **dadurch gekennzeichnet, daß** ein Edelmetallkatalysator, ausgewählt aus der Gruppe Palladium auf Aktivkohle, Kohle, Aluminiumoxid, Bariumcarbonat, Bariumsulfat, Calciumcarbonat oder Strontiumcarbonat, Platin auf Aktivkohle, Kohle oder Aluminiumoxid oder Rhodium auf Kohle oder Aluminiumoxid, eingesetzt wird.

4. Verfahren nach einem oder mehreren der Ansprüche 1 bis 3, **dadurch gekennzeichnet, daß** ein Wasserstoffakzeptor, ausgewählt aus der Gruppe Styrol, α-Methylstyrol, Stilben, Tolane, Zimtsäureester, Cyclohexen, Sauerstoff oder Sauerstoff-Gas-Gemisch, eingesetzt wird.

5. Verfahren nach einem oder mehreren der Ansprüche 1 bis 4, **dadurch gekennzeichnet, daß** zur Deacylierung ein primäres oder sekundäres Amin ausgewählt wird, dessen Siedepunkt zwischen 50 und 200°C liegt.

6. Verfahren nach einem oder mehreren der Ansprüche 1 bis 5, **dadurch gekennzeichnet, daß** die Reaktionen bei Temperaturen zwischen 50 und 200°C durchgeführt werden.

7. Verfahren nach einem oder mehreren der Ansprüche 1 bis 6, **dadurch gekennzeichnet, daß** die Reaktionen in einem hochsiedendem Lösungsmittel durchgeführt werden.

8. Verwendung von 2-Amino-benzo[b]thiophen-3-carbonsäuremethyl- oder -ethylester als Zwischenprodukt bei der Synthese von 4-[4-(3-Chlor-4-methoxy-benzylamino)-benzo[4,5]thieno[2,3-d]pyrimidin-2-yl]-cyclohexancarbonsäure.

9. Verfahren zur Herstellung von 4-[4-(3-Chlor-4-methoxy-benzylamino)-benzo[4,5]thieno[2,3-d]pyrimidin-2-yl]-cyclohexancarbonsäure oder einem der pharmazeutisch unbedenklichen Salze, **dadurch gekennzeichnet, daß** man in
Stufe a) 2-Acetylamino-tetrahydrobenzothiophen-3-carbonsäurealkylester gemäß einem oder mehreren der Ansprüche 1 bis 7 mit einer katalytischen Menge eines Edelmetallkatalysators in Gegenwart eines Wasserstoffakzeptors umsetzt und anschließend die acetylierte Aminogruppe durch Zugabe eines Amins zur Verbindung 2-Amino-benzo[b]thiophen-3-carbonsäurealkylester deacetyliert, in
Stufe b) 2-Amino-benzo[b]thiophen-3-carbonsäurealkylester durch Reaktion mit 4-Cyano-cyclohexancarbonsäurealkylester cyclisiert, in
Stufe c) die Hydroxygruppe der Verbindung 4-(4-Hydroxybenzo[4,5]thieno[2,3-d]pyrimidin-2-yl)-cyclohexancarbonsäurealkylester aus Stufe b) mit einem Chlorierungsmittel chloriert, in
Stufe d) die Verbindung 4-(4-Chlor-benzo[4,5]thieno[2,3-d]pyrimidin-2-yl)-cyclohexancarbonsäurealkylester aus Stufe c) mit 3-Chlor-4-methoxybenzylamin zu dem Ester 4-[4-(3-Chlor-4-methoxybenzylamino)-benzo[4,5]thieno[2,3-d]pyrimidin-2-yl]-cyclohexancarbonsäurealkylester umsetzt, in
Stufe e) den Ester aus Stufe d) verseift und in
Stufe f) die freie Säure in ein pharmakologisch unbedenkliches Salz überführt.

10. Verwendung von 2-Amino-benzo[b]thiophen-3-carbonsäuremethyloder -ethylester als Zwischenprodukt bei der Synthese von 4-[4-(3-Chlor-4-hydroxy-benzylamino)-benzo[4,5]thieno[2,3-d]pyrimidin-2-yl]-cyclohexancarbonsäure.

11. Verfahren zur Herstellung von 4-[4-(3-Chlor-4-hydroxy-benzylamino)-benzo[4,5]thieno[2,3-d]pyrimidin-2-yl]-cyclohexancarbonsäure oder einem der pharmazeutisch unbedenklichen Salze, **dadurch gekennzeichnet, daß** man in
Stufe a) 2-Acetylamino-tetrahydrobenzothiophen-3-carbonsäurealkylester gemäß einem oder mehreren der Ansprüche 1 bis 7 mit einer katalytischen Menge eines Edelmetallkatalysators in Gegenwart eines Wasserstoffakzeptors umsetzt und anschließend die acetylierte Aminogruppe durch Zugabe eines Amins zur Verbindung 2-Amino-benzo[b]thiophen-3-carbonsäurealkylester deacetyliert, in
Stufe b) 2-Amino-benzo[b]thiophen-3-carbonsäurealkylester durch Reaktion mit 4-Cyano-cyclohexancarbonsäurealkylester cyclisiert, in
Stufe c) die Hydroxygruppe der Verbindung 4-(4-Hydroxybenzo[4,5]thieno[2,3-d]pyrimidin-2-yl)-cyclohexancarbonsäurealkylester aus Stufe b) mit einem Chlorierungsmittel chloriert, in
Stufe d) die Verbindung 4-(4-Chlor-benzo[4,5]thieno[2,3-d]pyrimidin-2-yl)-cyclohexancarbonsäurealkylester aus Stufe c) mit 3-Chlor-4-hydroxybenzylamin zu dem Ester 4-[4-(3-Chlor-4-hydroxybenzylamino)-benzo[4,5]thieno[2,3-d]pyrimidin-2-yl]-cyclohexancarbonsäurealkylester umsetzt, in
Stufe e) den Ester aus Stufe d) verseift und in
Stufe f) die freie Säure in ein pharmakologisch unbedenkliches Salz überführt.

## Claims

1. Process for the preparation of benzo-fused heterocyclic compounds of the general formula I 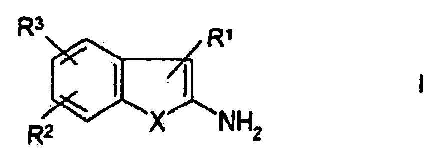 in which
X denotes S, O or NH,
R¹ denotes CN, NO₂, Ac, COAr, COOAr, COOH, COOA or CONR⁴R⁵,
R² and R³ each, independently of one another, denote H, A, NO₂, CN, OH, OA or Ac,
R⁴ and R⁵ each, independently of one another, denote H, A, Ar or Ac,
R⁴ and R⁵ together also denote -(CH₂)-(CH₂)ₙ-(CH₂)-,
A denotes alkyl having 1-6 C atoms,
Ac denotes acyl having 1-6 C atoms,
Ar denotes phenyl which is unsubstituted or substituted by A, NO₂, CN, OH or OA,
n denotes 2, 3 or 4,
by reaction of tetrahydrobenzo-fused heterocyclic compounds of the formula II 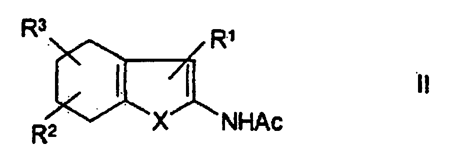 in which
X denotes S, O or NH,
R¹ denotes CN, NO₂, Ac, COAr, COOAr, COOH, COOA or CONR⁴R⁵,
R² and R³ each, independently of one another, denote H, A, NO₂, CN, OH, OA or Ac,
R⁴ and R⁵ each, independently of one another, denote H, A, Ar or Ac,
R⁴ and R⁵ together also denote -(CH₂)-(CH₂)ₙ-(CH₂)-,
A denotes alkyl having 1-6 C atoms,
Ac denotes acyl having 1-6 C atoms,
Ar denotes phenyl which is unsubstituted or substituted by A, NO₂, CN, OH, OA,
n denotes 2, 3 or 4,
with a catalytic amount of a noble-metal catalyst in the presence of a hydrogen acceptor and subsequent deacylation of the acylated amino group by addition of an amine.

2. Process according to Claim 1 for the preparation of methyl 2-aminobenzo[b]thiophene-3-carboxylate or ethyl 2-aminobenzo[b]thiophene-3-carboxylate.

3. Process according to Claim 1 or 2, **characterised in that** a noble-metal catalyst selected from the group consisting of palladium on activated carbon, carbon, aluminium oxide, barium carbonate, barium sulfate, calcium carbonate or strontium carbonate, platinum on activated carbon, carbon or aluminium oxide or rhodium on carbon or aluminium oxide is employed.

4. Process according to one or more of Claims 1 to 3, **characterised in that** a hydrogen acceptor selected from the group consisting of styrene, α-methylstyrene, stilbene, tolans, cinnamic acid esters, cyclohexene, oxygen or oxygen/gas mixture is employed.

5. Process according to one or more of Claims 1 to 4, **characterised in that**, for the deacylation, a primary or secondary amine is selected whose boiling point is between 50 and 200°C.

6. Process according to one or more of Claims 1 to 5, **characterised in that** the reactions are carried out at temperatures between 50 and 200°C.

7. Process according to one or more of Claims 1 to 6, **characterised in that** the reactions are carried out in a high-boiling solvent.

8. Use of methyl or ethyl 2-aminobenzo[b]thiophene-3-carboxylate as intermediate in the synthesis of 4-[4-(3-chloro-4-methoxybenzylamino)-benzo[4,5]thieno[2,3-d]pyrimidin-2-yl]cyclohexanecarboxylic acid.

9. Process for the preparation of 4-[4-(3-chloro-4-methoxybenzylamino)-benzo[4,5]thieno[2,3-d]pyrimidin-2-yl]cyclohexanecarboxylic acid or one of the pharmaceutically acceptable salts, **characterised in that** in
step a) alkyl 2-acetylaminotetrahydrobenzothiophene-3-carboxylate according to one or more of Claims 1 to 7 is reacted with a catalytic amount of a noble-metal catalyst in the presence of a hydrogen acceptor, and subsequently the acetylated amino group is deacetylated by addition of an amine to give the compound alkyl 2-aminobenzo[b]thiophene-3-carboxylate, in
step b) alkyl 2-aminobenzo[b]thiophene-3-carboxylate is cyclised by reaction with alkyl 4-cyanocyclohexanecarboxylate, in
step c) the hydroxyl group of the compound alkyl 4-(4-hydroxybenzo[4,5]thieno[2,3-d]pyrimidin-2-yl)cyclohexanecarboxylate from step b) is chlorinated using a chlorinating agent, in
step d) the compound alkyl 4-(4-chlorobenzo[4,5]thieno[2,3-d]-pyrimidin-2-yl)cyclohexanecarboxylate from step c) is reacted with 3-chloro-4-methoxybenzylamine to give the ester alkyl 4-[4-(3-chloro-4-methoxybenzylamino)benzo[4,5]thieno[2,3-d]pyrimidin-2-yl]cyclohexanecarboxylate, in
step e) the ester from step d) is saponified, and in
step f) the free acid is converted into a pharmacologically acceptable salt.

10. Use of methyl or ethyl 2-aminobenzo[b]thiophene-3-carboxylate as intermediate in the synthesis of 4-[4-(3-chloro-4-hydroxybenzylamino)-benzo[4,5]thieno[2,3-d]pyrimidin-2-yl]cyclohexanecarboxylic acid.

11. Process for the preparation of 4-[4-(3-chloro-4-hydroxybenzylamino)-benzo[4,5]thieno[2,3-d]pyrimidin-2-yl]cyclohexanecarboxylic acid or one of the pharmaceutically acceptable salts, **characterised in that** in
step a) alkyl 2-acetylaminotetrahydrobenzothiophene-3-carboxylate according to one or more of Claims 1 to 7 is reacted with a catalytic amount of a noble-metal catalyst in the presence of a hydrogen acceptor, and subsequently the acetylated amino group is deacetylated by addition of an amine to give the compound alkyl 2-aminobenzo[b]thiophene-3-carboxylate, in
step b) alkyl 2-aminobenzo[b]thiophene-3-carboxylate is cyclised by reaction with alkyl 4-cyanocyclohexanecarboxylate, in
step c) the hydroxyl group of the compound alkyl 4-(4-hydroxybenzo[4,5]thieno[2,3-d]pyrimidin-2-yl)cyclohexanecarboxylate from step b) is chlorinated using a chlorinating agent, in
step d) the compound alkyl 4-(4-chlorobenzo[4,5]thieno[2,3-d]-pyrimidin-2-yl)cyclohexanecarboxylate from step c) is reacted with 3-chloro-4-hydroxybenzylamine to give the ester alkyl 4-[4-(3-chloro-4-hydroxybenzylamino)benzo[4,5]thieno[2,3-d]pyrimidin-2-yl]cyclohexanecarboxylate, in
step e) the ester from step d) is saponified, and in
step f) the free acid is converted into a pharmacologically acceptable salt.

## Revendications

1. Procédé pour la préparation de composés hétérocycliques à fusion benzo de la formule générale I 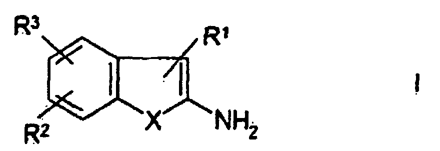 dans laquelle
X représente S, O ou NH,
R¹ représente CN, NO₂, Ac, COAr, COOAr, COOH, CODA ou CONR⁴R⁵,
R² et R³ chacun indépendamment l'un de l'autre, représentent H, A, NO₂, CN, OH, OA ou Ac,
R⁴ et R⁵ chacun indépendamment l'un de l'autre, représentent H, A, Ar ou Ac,
R⁴ et R⁵ ensemble représentent également -(CH₂)-(CH₂)ₙ-(CH₂)-,
A représente alkyle comportant 1-6 atomes C,
Ac représente acyle comportant 1-6 atomes C,
Ar représente phényle qui est non substitué ou substitué par A, NO₂, CN, OH ou OA,
n représente 2, 3 ou 4,
en faisant réagir des composés hétérocycliques à fusion tétrahydrobenzo de la formule II 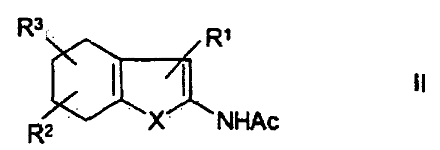 dans laquelle
X représente S, O ou NH,
R¹ représente CN, NO₂, Ac, COAr, COOAr, COOH, COOA ou CONR⁴R⁵,
R² et R³ chacun indépendamment l'un de l'autre, représentent H, A, NO₂, CN, OH, OA ou Ac,
R⁴ et R⁵ chacun indépendamment l'un de l'autre, représentent H, A, Ar ou Ac,
R⁴ et R⁵ ensemble représentent également -(CH₂)-(CH₂)ₙ-(CH₂)-,
A représente alkyle comportant 1-6 atomes C,
Ac représente acyle comportant 1-6 atomes C,
Ar représente phényle qui est non substitué ou substitué par A, NO₂, CN, OH, OA,
n représente 2, 3 ou 4,
avec une quantité de catalytique d'un catalyseur à métal noble en présence d'un accepteur d'hydrogène et ensuite une désacétylation du groupe amino acylaté par addition d'un amine.

2. Procédé selon la revendication 1 pour la préparation de 2-aminobenzo[b]thiophène-3-carboxylate de méthyle ou 2-aminobenzo[b]thiophène-3-carboxylate d'éthyle.

3. Procédé selon la revendication 1 ou 2, **caractérisé en ce qu'**un catalyseur à métal noble choisi parmi le groupe comprenant palladium sur carbone activé, carbone, oxyde d'aluminium, carbonate de baryum, sulfate de baryum, carbonate de calcium ou carbonate de strontium, platine sur carbone activé, carbone ou oxyde d'aluminium ou rhodium sur carbone ou oxyde d'aluminium est employé.

4. Procédé selon l'une ou plusieurs des revendications 1 à 3, **caractérisé en ce qu'**un accepteur d'hydrogène choisi parmi le groupe comprenant styrène, α-méthylstyrène, stilbène, tolans, esters d'acide cinnamique, cyclohexène, oxygène ou mélange oxygène/gaz est employé.

5. Procédé selon l'une ou plusieurs des revendications 1 à 4, **caractérisé en ce que**, pour la désacétylation, un amine primaire ou secondaire est choisi de telle sorte que son point d'ébullition soit entre 50 et 200°C.

6. Procédé selon l'une ou plusieurs des revendications 1 à 5, **caractérisé en ce que** les réactions sont mises en oeuvre à une température entre 50 et 200°C.

7. Procédé selon l'une ou plusieurs des revendications 1 à 6, **caractérisé en ce que** les réactions sont mises en oeuvre dans un solvant à haut point d'ébullition.

8. Utilisation de 2-aminobenzo[b]thiophène-3-carboxylate de méthyle ou d'éthyle en tant qu'intermédiaire dans la synthèse d'acide 4-[4-(3-chloro-4-méthoxybenzylamino)benzo[4,5]thiéno[2,3-d]pyrimidine-2-yl]cyclohexanecarboxylique.

9. Procédé pour la préparation d'acide 4-[4-(3-chloro-4-méthoxybenzylamino)benzo[4,5]thiéno[2,3-d]pyrimidine 2-yl]cyclohexanecarboxylique ou l'un de ses sels acceptables pharmaceutiquement, **caractérisé en ce que**,dans
l'étape a) 2-acétylaminotétrahydrobenzothiophène-3-carboxylate d'alkyle selon l'une ou plusieurs des revendications 1 à 7 est amené à réagir avec une quantité catalytique d'un catalyseur à métal noble en présence d'un accepteur d'hydrogène et ensuite, le groupe amino acétylaté est désacétylation par addition d'un amine pour donner le composé 2-aminobenzo[b]thiophène-3-carboxylate d'alkyle, dans
l'étape b) 2-aminobenzo[b]thiophène-3-carboxylate d'alkyle est cyclisé par réaction avec 4-cyanocyclohexanecarboxylate d'alkyle, dans
l'étape c) le groupe hydroxyle du composé 4-(4-hydroxybenzo[4,5]-thiéno[2,3-d]pyrimidine-2-yl)cyclohexanecarboxylate d'alkyle à partir de l'étape b) est chloré en utilisant un agent de chloration, dans
l'étape d) le composé 4-(4-chlorobenzo[4,5]thiéno[2,3-d]pyrimidine-2-yl)cyclohexanecarboxylate d'alkyle issu de l'étape c) est amené à réagir avec 3-chloro-4-méthoxybenzylamine pour donner l'ester 4-[4-(3-chloro-4-méthoxybenzylamino)benzo[4,5]thiéno[2,3-d]pyrimidine-2-yl]-cyclohexanecarboxylate d'alkyle, dans
l'étape e) l'ester issu de l'étape d) est saponifié, et dans
l'étape f) l'acide libre est converti selon un sel acceptable pharmacologiquement.

10. Utilisation de 2-aminobenzo[b]thiophène-3-carboxylate de méthyle ou d'éthyle en tant qu'intermédiaire dans la synthèse d'acide 4-[4-(3-chloro-4-hydroxybenzylamino)benzo[4,5]thiéno[2,3-d]pyrimidine-2-yl]-cyclohexanecarboxylique.

11. Procédé pour la préparation d'acide 4-[4-(3-chloro-4-hydroxybenzylamino)benzo[4,5]thiéno[2,3-d]pyrimidine-2-yl]cyclohexanecarboxylique ou l'un de ses sels acceptables pharmaceutiquement, **caractérisé en ce que**, dans
l'étape a) 2-acétylaminotétrahydrobenzothiophène-3-carboxylate d'alkyle selon l'une ou plusieurs des revendications 1 à 7 est amené à réagir avec une quantité catalytique d'un catalyseur à métal noble en présence d'un accepteur d'hydrogène et ensuite, le groupe amino acétylaté est désacétylation par addition d'un amine pour donner le composé 2-aminobenzo[b]thioph-ne-3-carboxylate d'alkyle, dans
l'étape b) 2-aminobenzo[b]thiophène-3-carboxylate d'alkyle est cyclisé par réaction avec 4-cyanocyclohexanecarboxylate d'alkyle, dans
l'étape c) le groupe hydroxyle du composé 4-(4-hydroxybenzo[4,5]-thiéno[2,3-d]pyrimidine-2-yl)cyclohexanecarboxylate d'alkyle issu de l'étape b) est chloré en utilisant un agent de chloration, dans
l'étape d) le composé 4-(4-chlorabenzo[4,5]thiéno[2,3-d]pyrimidine-2-yl)cyclohexanecarboxylate d'alkyle issu de l'étape c) est amené à réagir avec 3-chloro-4-hydroxybenzylamine pour donner l'ester 4-[4-(3-chloro-4-hydroxybenzylamino)benzo[4,5]thiéno[2,3-d]pyrimidine-2-yl]-cyclohexanecarboxylate d'alkyle, dans
l'étape e) l'ester issu de l'étape d) est saponifié, et dans
l'étape f) l'acide libre est converti selon un sel acceptable pharmacologiquement.
